# EUROPÄISCHE PATENTSCHRIFT

(11) **EP 1 776 050 B1**
(45) Veröffentlichungstag und Bekanntmachung des Hinweises auf die Patenterteilung: **31.07.2013**
(21) Anmeldenummer: 05777799.7
(22) Anmeldetag: 04.08.2005
(51) Int. Cl.: A61B 17/22

(54) **EINRICHTUNG ZUR KONTROLLE DER LAGE DES FOKUS EINES STOSSWELLENKOPFES**
DEVICE FOR VERIFYING THE FOCAL POSITION OF A SHOCK HEAD
DISPOSITIF POUR COMMANDER LA POSITION DE LA FOCALISATION D'UNE TETE A ONDES DE CHOC

(30) Priorität: 10.08.2004 DE 102004038848
(43) Veröffentlichungstag der Anmeldung: 25.04.2007
(73) Patentinhaber: Siemens Aktiengesellschaft, 80333 München (DE)
(72) Erfinder: MEINERT, Christian, 91080 Marloffstein (DE); BLESEL, Peter, 91083 Baiersdorf (DE); HOFMANN, Kirsten, 91052 Erlangen (DE)
(86) Internationale Anmeldenummer: PCT/EP2005/053828
(87) Internationale Veröffentlichungsnummer: WO 2006/018386

(56) Entgegenhaltungen:
- DE-A1- 19 640 050
- DE-U1- 8 914 437
- DE-U1- 29 823 797
- US-A- 4 819 638
- US-A- 5 070 861
- US-A- 5 240 000
- US-A- 5 465 625

## Beschreibung

Die Erfindung bezieht sich auf eine Einrichtung zur Kontrolle der Lage des Fokus eines Stoßwellenkopfes mit einem medizinischen Bildgebungssystem.

Bei der extrakorporalen Stoßwellenlithotripsie ist für den Behandlungserfolg eine genaue Positionierung des Fokus der von einem Stoßwellenkopfe erzeugten fokussierten UltraschallStoßwelle notwendig. Die Lage des Fokus muss dabei im Bildkoordinatensystem des zur Positionierung des Stoßwellenkopfes verwendeten medizinischen Bildgebungssystems bekannt sein, um eine genaue Ausrichtung des Fokus auf ein zu zerstörendes Konkrement zu ermöglichen. Zur Kontrolle und Justage der Lage des Fokus ist es bekannt, ein sogenanntes Fokusphantom zu verwenden, das am Stoßwellenkopf angeordnet wird und am Ort des Fokus einen Marker in Form einer metallischen Kugel trägt. Dieser Marker wird mit dem Bildgebungssystem abgebildet, so dass die Position des Fokus unmittelbar mit Hilfe des Bildgebungssystems betrachtet werden kann. Die Genauigkeit, mit der mit Hilfe eines solchen Fokusphantoms die tatsächliche Lage des Fokus simuliert und damit kontrolliert werden kann, ist für die Genauigkeit der Positionierung des Fokus und somit für den Therapieerfolg von entscheidender Bedeutung. Diese Genauigkeit wird dabei im wesentlichen bestimmt durch Toleranzen der die Lage des Markers relativ zum Stoßwellenkopf festlegenden Bauteile.

Dokument DE-U-29823797 offenbart eine Einrichtung zur Kontrolle der Lage des Fokus eines Stosswellenkopies mit einem medizinischen Bildgebungssystem, mit einem auf einem Stosswellenerzeuger unmittelbar aufsetzbaren und in einer vorbestimmten Position fixierbaren Traggestell, an dem ortsfest ein im Bildgebungssystem abbildbarer Marker angeordnet ist, dessen Position bei fixiertem Traggestell mit der Lage des Fokus übereinstimmt. Bei der aus US-A-4819638 bekannten Einrichtung, ist das eine Testeinrichtung aufnehmende Traggestell nicht unmittelbar auf die akustische Linse aufgesetzt, sondern auf einem die Linse aufnehmenden Gehäuse aufgeschoben.

Der Erfindung liegt nun die Aufgabe zu Grunde, eine Einrichtung zur Kontrolle der Lage des Fokus eines Stoßwellenkopfes anzugeben, die systembedingt eine möglichst geringe Anzahl von fehlerverursachenden Toleranzen aufweist.

Die genannte Aufgabe wird gemäß der Erfindung gelöst mit einer Einrichtung mit den Merkmalen des Patentanspruches 1. Gemäß diesen Merkmalen ist bei einer Einrichtung zur Kontrolle der Lage des Fokus eines Stoßwellenkopfes mit einem medizinischen Bildgebungssystem ein auf eine akustische Linse des Stoßwellenkopfes unmittelbar aufsetzbares und in einer vorbestimmten Position fixierbares Traggestell vorgesehen, an dem ortsfest ein im Bildgebungssystem abbildbarer Marker angeordnet ist, dessen Position bei fixiertem Traggestell mit der Lage des Fokus übereinstimmt. Da das Traggestell unmittelbar auf die akustische Linse und somit auf das die Lage des Fokus der Ultraschallstoßwelle bestimmende Bauteil des Stoßwellenkopfes aufgesetzt wird, ist systembedingt die Anzahl der Bauteile zwischen der die Fokuslage bestimmenden Linse und dem Marker reduziert. Dadurch ist die entsprechend der Anzahl der Bauteile sich ergebende Summe der unvermeidbaren fertigungstechnischen Toleranzen, die zu einer Abweichung der Position des Markers von der tatsächlichen Lage des Fokus führen können, verringert.

In einer vorteilhaften Ausführungsform ist der Marker auf einer Längsmittenachse des Traggestells angeordnet und das Traggestell ist mit seiner Mittenlängsachse koaxial zur Mittenachse der Linse in einer Mehrzahl von Drehpositionen auf die Linse aufsetzbar. Dadurch ist eine einfache Kontrolle der Genauigkeit der Einrichtung möglich. Bleibt nämlich die Lage des Markers im Bild des Bildgebungssystems bei unterschiedlichen Drehpositionen des Traggestells unverändert, ist dies ein Indiz dafür, dass Fokusphantom und die zugehörigen geometrischen Verhältnisse am Stoßwellenkopf in einem einwandfreien Zustand sind.

In einer weiteren vorteilhaften Ausgestaltung weist die Linse an ihrem Rand eine ringförmige Auflagefläche für das Traggestell auf. Dies ermöglicht auf eine Weise eine Lagerung des Traggestells auf der Linse in unterschiedlichen Drehpositionen. Eine solche rotationssymmetrisch um die Mittenachse der Linse sich erstreckende Auflagefläche ist außerdem fertigungstechnisch einfach herzustellen.

Alternativ hierzu können am Rand der Linse auch eine Mehrzahl von räumlich voneinander getrennten Auflageflächen für das Traggestell angeordnet sein.

Insbesondere weist das Traggestell eine Mehrzahl von symmetrisch um die Mittenlängsachse des Traggestells angeordnete Stützflächen, vorzugsweise drei Stützflächen auf, die auf der Auflagefläche oder den Auflageflächen aufliegen. Durch eine solche Dreipunktlagerung ist ein Verkippen verhindert und die Genauigkeit der Positionierung des Markers erhöht.

Wenn das Traggestell mit einer ringförmig umlaufenden Stützfläche versehen ist, ist ein besonders sicherer Sitz des Traggestells auf der Linse gewährleistet.

In einer weiteren bevorzugten Ausführungsform sind am Traggestell eine Mehrzahl von radial in das Innere des Traggestells weisenden Haltestiften angeordnet, die durch Drehbewegung des Traggestells um seine Mittenlängsachse in korrespondierende Nuten am Umfang der Linse einführbar sind und das Traggestell axial fixieren. Dadurch wird ein weitgehend spielfreies Anlegen des Traggestells auf der Linse sichergestellt.

Insbesondere weist das Traggestell ein einstückiges Basisteil auf, mit dem es auf der Linse aufliegt, an dem eine den Marker enthaltende ebenfalls einstückige Halteplatte fixiert ist. Dadurch ist die Anzahl der Bauteile und damit die Anzahl der sich addierenden Toleranzen reduziert und die Positioniergenauigkeit des Markers erhöht.

Wenn sich der Marker in einem am Traggestell angeordneten Schutzgehäuse befindet, ist dieser vor einer unbeabsichtigten Beschädigung geschützt.

Insbesondere besteht das Schutzgehäuse aus Kunststoff und kann mit einer Flüssigkeit befüllt werden. Dadurch ist die Einrichtung auch für einen Stoßwellenlithotripter geeignet, bei dem zur Bildgebung ein Ultraschallbildgebungssystem verwendet wird.

Wenn außerdem das Schutzgehäuse eine kugelförmige Kuppel aufweist, kann die Einrichtung sowohl bei einer Inline- als auch bei einer Outline-Ultraschallbildgebung verwendet werden.

Durch die Verwendung eines zumindest in einem Teil optisch transparenten Schutzgehäuses kann der Marker außerdem einer Sichtkontrolle unterzogen werden.

Zur weiteren Erläuterung der Erfindung wird auf das Ausführungsbeispiel der Zeichnung verwiesen. Es zeigen:
- FIG 1: eine Einrichtung gemäß der Erfindung mit einem auf einen Stoßwellenkopf aufgesetzten Traggestell in einer perspektivischen Darstellung,
- FIG 2: das Traggestell in einem Längsschnitt.

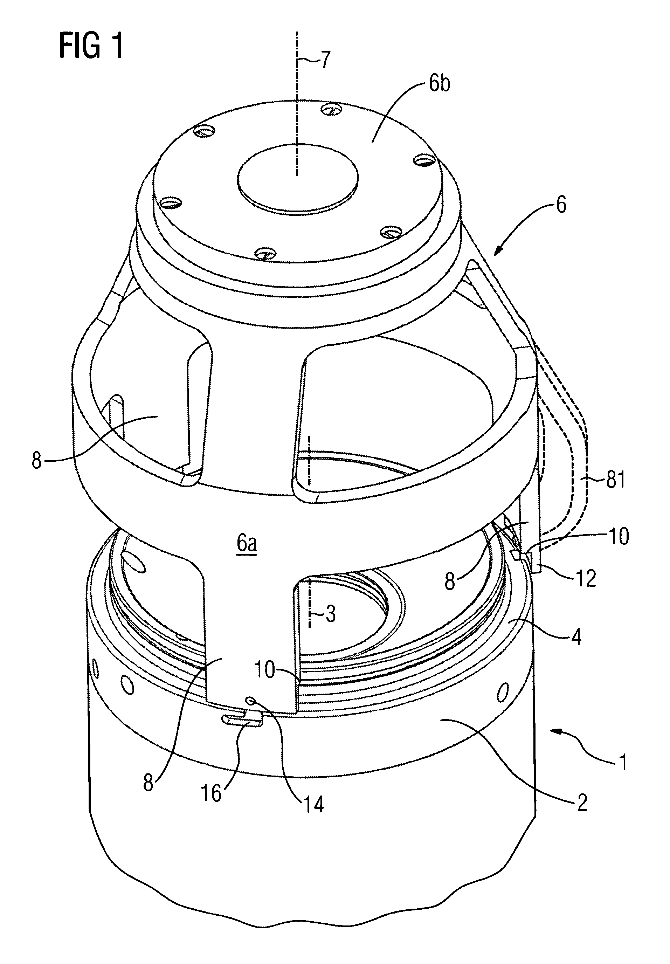

Gemäß FIG 1 ist bei einer Einrichtung gemäß der Erfindung eine akustische Linse 2 eines Stoßwellenkopfes 1 an ihrem Rand mit einer ringförmig um ihre Mittenachse 3 (akustische Achse) umlaufenden Auflagefläche 4 in Form einer Schulter versehen, auf der ein annähernd hohlzylindrisches Traggestell 6 aufliegt. In dem als sogenanntes Fokusphantom dienenden Traggestell 6 ist ortsfest ein in einem medizinischen Bildgebungssystem abbildbarer, in der Darstellung der FIG nicht sichtbarer Marker angeordnet. Das Traggestell 6 besteht aus einem einstückigen Basisteil 6a und einer in ihm zentrisch gelagerten ebenfalls einstückigen Halteplatte 6b für den Marker, die an dem der akustischen Linse 2 gegenüberliegenden freien Ende des Traggestells 6 am Basisteil 6a fixiert ist. Im Ausführungsbeispiel weist das Basisteil 6a drei symmetrisch um eine Mittenlängsachse 7 des Traggestells 6 angeordnete, sich in Richtung dieser Mittenlängsachse 7 erstreckende Stützstreben 8 auf, mit denen es im Montageendzustand - in der FIG ist das Traggestell 6 unmittelbar vor dem Aufsetzen auf die akustische Linse 2 dargestellt - auf der akustischen Linse 2 aufliegt. Die Stützstreben 8 sind jeweils an ihrem freien Ende auf ihrer Innenseite mit einer Ausnehmung versehen, so dass eine innenliegende Schulter gebildet wird, die jeweils als Stützfläche 10 dient, mit der die Stützstreben 8 auf der Aufliegefläche 4 aufsitzen.

Die innenliegende Schulter wird von einem außenliegenden axialen, d.h. in Richtung der Mittenlängsachse 7 sich erstreckenden Überstand oder Fortsatz 12 begrenzt, der am äußeren Umfang der Linse 4 anliegt und das Traggestell 6 radial fixiert. An diesem axialen Fortsatz 12 ist ein radial nach innen vorstehender Haltezapfen 14 angeordnet, der beim Aufsetzen auf die Linse 2 in eine am Rand der Linse 2 angeordnete axiale, sich bis zur Auflagefläche 4 erstreckende Nut 16a eingeführt wird. Die Nut 16a mündet in eine dazu annähernd quer, d.h. am Außenumfang der Linse 2 in Umfangsrichtung verlaufende Nut 16b, so dass das Tragegestell 6 durch Verdrehen um seine Mittenlängsachse 7 axial in einer vorbestimmten Drehposition fixiert wird. Die Nut 16b verläuft dabei unter einer leichten Schräge zur Auflagefläche 4, so dass bei einem Verdrehen des Traggestells 6 die Stützfläche 10 auf die Auflagefläche 4 gepresst wird und spielfrei auf dieser aufliegt. Durch die axiale und radiale Fixierung des Traggestells 6 ist sichergestellt, dass sich der in der Halteplatte 6b ortsfest angeordnete Marker im Montageendzustand stets in derselben Position relativ zur Linse 2 befindet. Im Ausführungsbeispiel fallen dann Mittenachse 3 der Linse und Mittenlängsachse 7 des Tragegestells 6 zusammen. Der Marker ist mittig, d.h. auf der Mittenlängsachse 7 innerhalb des Traggestells 6 angeordnet. Dadurch kann das Traggestell 6 in unterschiedlichen Drehpositionen auf der Linse 2 angeordnet werden. Dies ermöglicht eine einfache Kontrolle der Einrichtung. Hierzu wird zunächst das Traggestell 6 in einer Position aufgesetzt und mit dem Bildgebungssystem ein den Marker enthaltendes Bild erzeugt und gespeichert. Anschließend wird das Traggestell 6 in einer zweiten Drehposition aufgesetzt und erneut ein Bild erzeugt. Im Falle einer Dejustage der Einrichtung, d.h. der die Position des Markers festlegenden Teile des Traggestells 6 und den korrespondierenden Aufnahmen an der Linse 2 des Stoßwellenkopfes 1, verändert der Marker seine Position im Bild. Bleibt er in allen drei Drehpositionen ortsfest, ist dies ein Indiz dafür, dass die Einrichtung, d.h. Traggestell 6 und die entsprechenden Aufnahmen am Stoßwellenkopf 1 einwandfrei ist und noch der Auslieferungsqualität entspricht.

Alternativ zu der in der FIG dargestellten Ausführungsform, bei der das Traggestell 6 drei Stützstreben 8 und somit auch drei durch die jeweiligen Schultern gebildete Stützflächen 10 aufweist, ist es auch möglich, das Traggestell mit einer einzigen ringförmig umlaufenden Stützfläche zu versehen.

In der FIG ist außerdem eine alternative Ausgestaltung der Stützstreben 8 gestrichelt eingezeichnet, bei der diese eine radiale Ausformung 81 aufweisen. Durch eine solche Ausformung 81 wird ausreichend Platz für einen auf der Linse 2 angeordneten und aus Gründen der Übersichtlichkeit nicht dargestellten Koppelbalg geschaffen.

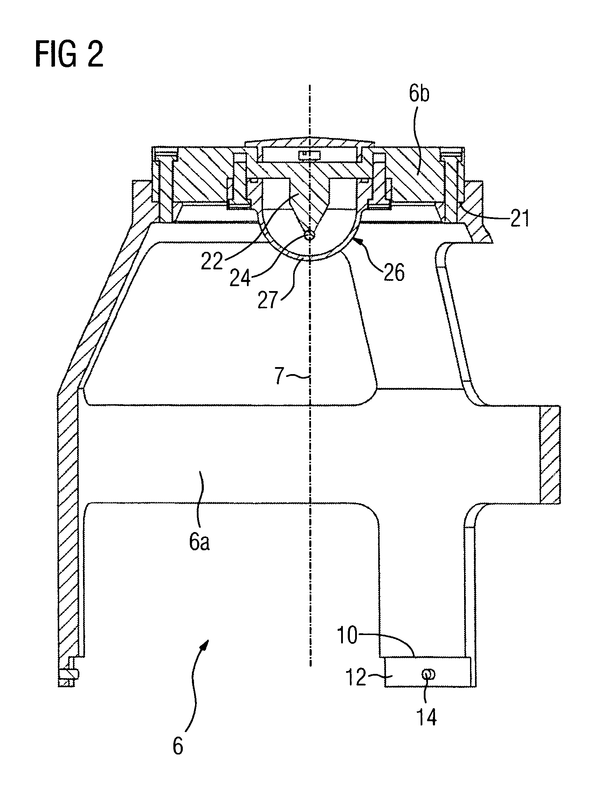

Gemäß FIG 2 hat die Halteplatte 6b eine annähernd kreisscheibenförmige Gestalt und sitzt auf einer von einer innen umlaufenden Ausnehmung gebildeten ringförmigen Schulter 21 des Basisteils 6a auf. In der Mitte der Halteplatte 6b ist ein axial ins Innere des Traggestells 6 hineinragender Dorn 22 angeformt, auf dessen auf der Mittenlängsachse 7 liegenden Spitze ein Marker 24 in Form einer metallischen Kugel aufgeklebt ist. Der Dorn 22 ist innerhalb eines Schutzgehäuses 26 angeordnet, das auf die Innenseite der Halteplatte 6b aufgesetzt und mit diesem dicht verschraubt ist. Das Schutzgehäuse 26 weist eine ins Innere des Traggestells 6 ragende kugelförmige Kuppel 27 auf, und sein Innenraum kann mit Wasser gefüllt werden, um eine Verwendung des Traggestells 6 bei einem Lithotripter mit einem Ultraschallbildgebenden System zu ermöglichen. Das Schutzgehäuse 26 besteht aus einem optisch transparenten Kunststoff, so dass auch eine visuelle Kontrolle des Markers 24 möglich ist. Alternativ hierzu ist es auch möglich, den Marker 24 in einen massiven, für Ultraschall transparenten Körper einzubetten.

Das gesamte Traggestell 6 besteht aus Kunststoff und ist nur aus dem einstückigen Basisteil 6a und der ebenfalls einstückigen Halteplatte 6b, die beide Spritzgussteile sind, zusammengesetzt. Alternativ hierzu kann auch das gesamte Traggestell aus einem einzigen Bauteil gefertigt werden. Durch die Verwendung höchstens zweier Bauteile wird die Anzahl der unvermeidlichen fertigungstechnischen Toleranzen verringert. Die Anzahl der durch verschiedene Bauteile vorhandenen Toleranzen, die in der Summe eine Abweichung der Position des Markers 24 von der Sollposition bewirken können, ist außerdem durch die unmittelbare Positionierung des Traggestells 6 auf der akustischen Linse 2 verringert.

## Patentansprüche

1. Stoßwellenkopf (1) mit einer Einrichtung zur Kontrolle der Lage des Fokus mit einem medizinischen Bildgebungssystem, mit einem auf eine akustische Linse (2) des Stoßwellenkopfes (1) unmittelbar aufsetzbaren und in einer vorbestimmten Position fixierbaren Traggestell (6), an dem ortsfest ein im Bildgebungssystem abbildbarer Marker (24) angeordnet ist, dessen Position bei fixiertem Traggestell (6) mit der Lage des Fokus übereinstimmt.

2. Stoßwellenkopf (1) nach Anspruch 1, bei dem der Marker (24) auf einer Mittenlängsachse (7) des Traggestells (6) angeordnet ist, und bei der das Traggestell (6) mit seiner Längsmittenachse (7) koaxial zur Mittenachse (3) der Linse (2) in einer Mehrzahl von Drehpositionen auf die Linse (2) aufsetzbar ist.

3. Stoßwellenkopf (1) nach Anspruch 1 oder 2, bei dem die Linse (2) an ihrem Rand eine ringförmige Auflagefläche (4) für das Traggestell (6) aufweist.

4. Stoßwellenkopf (1) nach Anspruch 3, bei dem das Traggestell (6) eine Mehrzahl von symmetrisch um die Mittenlängsachse (7) angeordnete Stützflächen (10) aufweist.

5. Stoßwellenkopf (1) nach Anspruch 4, bei dem drei Stützflächen (10) vorgesehen sind.

6. Stoßwellenkopf (1) nach Anspruch 3, bei dem das Traggestell (6) eine ringförmig umlaufende Stützfläche aufweist.

7. Stoßwellenkopf (1) nach einem der vorhergehenden Ansprüche, bei dem am Traggestell (6) eine Mehrzahl von radial in das Innere des Traggestells (6) weisenden Haltestiften (14) angeordnet sind, die durch Drehbewegung des Traggestells (6) um seine Mittenlängsachse (7) in korrespondierende Nuten (16a, b) am Umfang der Linse (2) einführbar sind und das Traggestell (6) axial fixieren.

8. Stoßwellenkopf (1) nach einem der vorhergehenden Ansprüche, bei dem das Traggestell (6) ein einstückiges Basisteil (6a) aufweist, mit dem es auf der Linse (2) aufliegt, an dem eine den Marker (24) enthaltende ebenfalls einstückige Halteplatte (6b) fixiert ist.

9. Stoßwellenkopf (1) nach einem der vorhergehenden Ansprüche, bei dem sich der Marker (24) in einem am Traggestell (6) angeordneten Schutzgehäuse (26) befindet.

10. Stoßwellenkopf (1) nach Anspruch 9, bei dem das Schutzgehäuse (26) aus Kunststoff besteht und mit einer Flüssigkeit befüllbar ist.

11. Stoßwellenkopf (1) nach Anspruch 10, bei dem das Schutzgehäuse (26) eine kugelförmige Kuppel (27) aufweist.

12. Stoßwellenkopf (1) nach einem der Ansprüche 9 bis 11, bei dem zumindest ein Teil des Schutzgehäuses (26) optisch transparent ist.

## Claims

1. Shockwave head (1) with a device for checking the position of the focus with a medical imaging system, with a support frame (6) which can be directly mounted onto an acoustic lens (2) of the shockwave head (1) and fixed in a predetermined position, on which support frame (6) a marker (24) which can be mapped in the imaging system is permanently arranged, the position of said marker (24) corresponding to the position of the focus when the support frame (6) is fixed.

2. Shockwave head (1) according to claim 1, wherein the marker (24) is arranged on a central longitudinal axis (7) of the support frame (6), and wherein the longitudinal central axis (7) of the support frame (6) can be mounted onto the lens (2) coaxially to the central axis (3) of the lens (2) in a plurality of rotational positions.

3. Shockwave (1) head according to claim 1 or 2, wherein the edge of the lens (2) has an annular bearing surface (4) for the support frame.

4. Shockwave head (1) according to claim 3, wherein the support frame (6) has a plurality of supporting surfaces (10) arranged symmetrically around the central longitudinal axis (7).

5. Shockwave head (1) according to claim 4, wherein three supporting surfaces (10) are provided.

6. Shockwave head (1) according to claim 3, wherein the support frame (6) has an annularly circumferential supporting surface.

7. Shockwave head (1) according to one of the preceding claims, wherein a plurality of retaining pins (14) pointing radially into the interior of the support frame (6) are arranged on the support frame (6), can be introduced through the rotary movement of the support frame (6) about its central longitudinal axis (7) into corresponding grooves (16a, b) on the circumference of the lens (2), and axially fix the support frame (6).

8. Shockwave head (1) according to one of the preceding claims, wherein the support frame (6) has a one-piece base part (6a), with which it rests on the lens (2), on which a likewise one-piece retaining plate (6b) containing the marker (24) is fixed.

9. Shockwave head (1) according to one of the preceding claims, wherein the marker (24) is located in a protective housing (26) arranged on the support frame (6).

10. Shockwave head (1) according to claim 9, wherein the protective housing (26) is made of plastic and can be filled with a liquid.

11. Shockwave head (1) according to claim 10, wherein the protective housing (26) has a spherical dome (27).

12. Shockwave head (1) according to one of claims 9 to 11, wherein at least part of the protective housing (26) is optically transparent.

## Revendications

1. Tête (1) à ondes de choc, ayant un dispositif de contrôle de la position du foyer par un système médical donnant une image, un bâti (6) support pouvant être mis directement sur une lentille (2) acoustique de la tête (1) à ondes de choc et pouvant être immobilisé dans une position déterminée à l'avance, bâti sur lequel est monté, à poste fixe, un marqueur (24) qui peut être reproduit dans le système donnant une image et dont la position coïncide, lorsque le bâti (6) support est immobilisé, avec la position du foyer.

2. Tête (1) à ondes de choc suivant la revendication 1, dans laquelle le marqueur (24) est disposé sur un axe (7) longitudinal médian du bâti (6) support et dans laquelle le bâti (6) support peut être mis, par son axe (7) médian longitudinal, coaxialement à l'axe (3) médian de la lentille (2) dans une multiplicité de positions en rotation sur la lentille (2).

3. Tête (1) à ondes de choc suivant la revendication 1 ou 2,
dans laquelle la lentille (4) a sur son bord une surface (4) annulaire d'appui du bâti (6) support.

4. Tête (1) à ondes de choc suivant la revendication 3, dans laquelle le bâti (6) support a une multiplicité de surfaces (10) d'appui, disposées symétriquement autour de l'axe (7) longitudinal médian.

5. Tête (1) à ondes de choc suivant la revendication 4, dans laquelle il est prévu trois surfaces (10) d'appui.

6. Tête (1) à ondes de choc suivant la revendication 3, dans laquelle le bâti (6) support a une surface d'appui annulaire faisant le tour.

7. Tête (1) à ondes de choc suivant l'une des revendications précédentes,
dans laquelle il est disposé sur le bâti (6) support une multiplicité de chevilles (14) de maintien, qui sont tournées radialement vers l'intérieur du bâti (6) support, qui, par un mouvement de rotation du bâti (6) support autour de son axe (7) longitudinal médian, peuvent venir dans des rainures (16a, b) correspondantes du pourtour de la lentille (2) et qui immobilisent axialement le bâti (6) support.

8. Tête (1) à ondes de choc suivant l'une des revendications précédentes,
dans laquelle le bâti (6) support a une partie (6a) de base d'un seul tenant, par laquelle il repose sur la lentille (2) et sur laquelle est immobilisée une plaque (6b) de maintien également d'un seul tenant comprenant le marqueur (24).

9. Tête (1) à ondes de choc suivant l'une des revendications précédentes,
dans laquelle le marqueur (24) se trouve dans un boîtier (26) de protection disposé sur le bâti (6) support.

10. Tête (1) à ondes de choc suivant la revendication 9, dans laquelle le boîtier (26) de protection est en matière plastique et peut être rempli d'un liquide.

11. Tête (1) à ondes de choc suivant la revendication 10, dans laquelle le boîtier (26) de protection a une coupole (27) sphérique.

12. Tête (1) à ondes de choc suivant l'une des revendications 9 à 11,
dans laquelle au moins une partie du boîtier (26) de protection est transparente optiquement.
